# EUROPEAN PATENT APPLICATION

(11) **EP 2 210 593 A2**
(43) Date of publication of application: **28.07.2010**
(21) Application number: 10151161.6
(22) Date of filing: 20.01.2010
(51) Int. Cl.: A61K 9/20, A61K 47/10, A61K 31/07

(54) **Tablettable formulations of vitamin A and derivatives thereof**

(30) Priority: 21.01.2009 EP 09151006
(71) Applicant: DSM IP Assets B.V., 6411 TE Heerlen (NL)
(72) Inventor: Leuenberger, Bruno H., 4310, Rheinfelden (CH); Schweikert, Loni, 4315, Zuzgen (CH)
(74) Representative: Steck, Melanie

(57) **Abstract**

The present invention is directed to the use of an antioxidant selected from the group consisting of 2,6-ditertbutyl-4-methylphenol, 2-tert-butyl-4-hydroxyanisol and mixtures thereof to increase the stability of gelatine-free formulations of vitamin A and its derivatives and to increase the stability of gelatine-free tablets based on such formulations.

The present invention is further directed to the formulations and the tablets themselves, as well as to a process preparing such formulations.

## Description

The present invention is directed to the use of the antioxidants 2,6-ditertbutyl-4-methylphenol (BHT) and/or 2-tert-butyl-4-hydroxyanisol (BHA) to increase the stability of gelatine-free formulations of vitamin A and its derivatives, as well as to increase the stability of vitamin A tablets based on these formulations.

The invention is further directed to a formulation of vitamin A which is suitable for pharmaceutical applications comprising vitamin A and/or a derivate thereof, a protective colloid and an antioxidant selected from the group consisting of 2,6-ditertbutyl-4-methylphenol (BHT), 2-tert-butyl-4-hydroxyanisol (BHA) and mixtures thereof, wherein the protective colloid is not gelatine, as well as to tablets based on such formulations and to a process for the manufacture of such formulations.

Vitamin A tablets based on gelatine-containing (fish gelatine, poultry gelatine, pork gelatine, beef gelatine) formulations of vitamin A are in general more stable than vitamin A tablets based on gelatine-free formulations of vitamin A. According to the growing awareness of allergenic issue there is, however, a need to avoid the use of gelatine. Thus, there is a need to find formulations, not based on gelatine, which may be used to prepare vitamin A tablets with increased stability.

This need is fulfilled by the formulations of the present invention. Such formulations of vitamin A according to the present invention are suitable for pharmaceutical applications and comprise vitamin A and/or a derivate thereof, a protective colloid and an antioxidant selected from the group consisting of BHT, BHA and mixtures thereof, wherein the protective colloid is not gelatine. Thus, the formulation itself does not contain gelatine and is therefore animal-free.

Preferred formulations according to the present invention comprise vitamin A and/or a derivate thereof, a protective colloid and a mixture of BHT and BHA (preferably a mixture of BHT and BHA in a weight-ratio of 1 : 1), wherein the protective colloid is not gelatine.

That means that this formulation and also the other formulations according to the present invention are gelatine free.

The total amount of the antioxidants BHT and/or BHA in the formulations of the present invention is preferably in the range of from 0.5 to 25 mg, more preferably in the range of from 1 to 15 mg, even more preferably in the range of from 2 to 15 mg, most preferably in the range of from 2 to 12 mg, per 100000 IUs (**I**nternational **U**nit**s**) of vitamin A in the formulation.

A further object of the present invention is a tablet comprising
i) a carrier selected from the group consisting of calcium carbonate, microcrystalline cellulose, magnesium stearate, maltodextrin, and mixtures thereof; and
ii) a formulation, wherein the formulation comprises vitamin A and/or a derivate thereof, a protective colloid and an antioxidant selected from the group consisting of BHT, BHA and mixtures thereof, wherein the protective colloid is not gelatine.

A preferred tablet according to the present invention comprises:
i) a carrier selected from the group consisting of calcium carbonate, microcrystalline cellulose, magnesium stearate, maltodextrin, and mixtures thereof; and
ii) a formulation, wherein the formulation comprises vitamin A and/or a derivate thereof, a protective colloid and a mixture of BHT and BHA (preferably a mixture of BHT and BHA in a weight-ratio of 1 : 1), wherein the protective colloid is not gelatine.

Preferably also no gelatine is used for the manufacture of the tablet. Thus, the formulations and the tablets of the present invention are gelatine-free, i.e. animal-free.

Surprisingly it was found that gelatine-free Vitamin A tablets that contain BHT and/or BHA as antioxidants are more stable than gelatine-free Vitamin A tablets that contain another antioxidant such as e.g. tocopherol.

More surprisingly it was found that the mixture of the two antioxidants BHT and BHA leads to an increased stability of vitamin A tablets compared to the stability of vitamin A tablets that contain (a) different antioxidant/s such as e.g. tocopherol.

The tablets may be coated or non-coated with materials known to the person skilled in the art. The formulations according to the present invention are mixed with the carrier(s) and/or other excipient(s) in amounts usually taken for that purpose and formed into the desired tablet form using conventional techniques.

The term "vitamin A" in the context of the present invention encompasses vitamin A itself, as well as any derivative thereof. The term, thus, encompasses especially the vitamin A alcohol, as well as its derivatives such as vitamin A ester (e.g. vitamin A acetate, vitamin A palmitate, vitamin A propionate), vitamin A aldehyde, vitamin A ether and vitamin A acid. Preferred are Vitamin A esters, more preferred are vitamin A acetate and vitamin A palmitate, most preferred is Vitamin A acetate and especially the corresponding formulation having a Vitamin A acetate content of at least 1 weight-%, preferably of at least 5 weight-%, based on the total weight of the formulation. Such formulations usually have a Vitamin A activity in the range of from 250000 to 500000 International Units per g of the formulation.

Preferably the formulations of the present invention contain an amount of vitamin A and/or its derivatives in the range of from 25000 to 650000 IUs, preferably in the range of from 50000 to 500000 IUs, most preferably in the range of from 100000 to 500000 IUs, per g of the formulation.

"Increasing the stability of formulations/tablets" in the context of the present invention means prolonging the shelf life of formulations/tablets. Thus, the content of vitamin A in the formulations/tablets is at the same high content level or starting level (i.e. the level of vitamin A the formulation/tablet has just after preparation) for a longer period of time compared to the content of vitamin A in formulations/tablets that do not contain BHT and/or BHA.

The term "protective colloid" denotes in the scope of the present invention carbohydrates such as modified starch, e.g. an "OSA-starch" (starch sodium octenyl succinate), sugars (e.g. maltose (syrup)), dextrin, pectin, gum arabic, a modified plant gum, lignin derivatives (e.g. lignin sulfonate), milk protein such as e.g. casein, and vegetable protein (e.g. soy protein, rice protein, lupine protein, potato protein), as well as mixtures thereof.

If the formulations consist only of vitamin A and/or its derivatives, the antioxidant(s) BHT and/or BHA and the protective colloid, the amount of the protective colloid may be calculated easily by taking into account the amounts of vitamin A and/or its derivatives and the amounts of the antioxidant(s) given above, i.e. 100 weight-% minus (sum of amount of vitamin A (derivative) and amount of BHT/BHA).

If further ingredients (see below) such as fat-soluble excipient(s) and/or adjuvant(s), water-soluble excipient(s) and/or adjuvant(s), emulsifiers (preferably present in an amount of from 0-10 weight-%, based on the total weight of the formulation) or in case of beadlets (manufactured by a powder-catch process) coatings of e.g. corn starch (present in an amount of from 0-40 weight-%, preferably in an amount of from 0-25 weight-%, based on the total weight of the formulation) are present, the amount of the protective colloid may be correspondingly lower.

The formulations according to the present invention may further contain one or more water-soluble excipient(s) and/or adjuvant(s), one or more fat-soluble excipient(s) and/or adjuvant(s) and/or one or more emulsifier(s).

Examples of water-soluble excipients and/or adjuvants are monosaccharides, disaccharides, oligosaccharides and polysaccharides, glycerol and water-soluble antioxidants.

### Tablets according to the present invention

The formulations according to the present invention are especially suitable for the manufacture of tablets of vitamin A and its derivatives with the preferences as given above.

The term "suitable for pharmaceutical applications" in the context of the present invention means "suitable for the manufacture of tablets", whereby the tablets may be pharmaceuticals or dietary supplements.

The present invention is also directed to tablets comprising a formulation according to the present invention, as well as a carrier selected from the group consisting of calcium carbonate, microcrystalline cellulose, magnesium stearate, maltodextrin, and mixtures thereof. The tablets may be any pharmaceutical or dietary supplement that preferably contains Vitamin A or a derivative thereof, especially any pharmaceutical that contains Vitamin A acetate.

Preferably those tablets are multivitamin tablets and tablets comprising mineral salts and/or trace elements, as well as multivitamin tablets also containing mineral salts and/or trace elements.

Those multivitamin tablets may contain vitamin E, vitamin C, vitamin K, vitamin B1, vitamin B2, vitamin B6, vitamin B12, vitamin D, biotin, folic acid, niacin, niacin amide, pantothenic acid and/or pantothenate. Instead of these compounds also the corresponding derivatives and salts may be used.

Those mineral salts-tablets may contain calcium, phosphor, magnesium, potassium, iron, manganese, selenium, copper, chloride, molybdenum, chrome, zinc and/or iodine salts.

Especially preferred is the use of a formulation according to the present invention of Vitamin A for the manufacture of a tablet comprising other water- and fat-soluble vitamins such as vitamin E, vitamin C, vitamin K, vitamin B1, vitamin B2, vitamin B6, vitamin B12, vitamin D and their derivatives or salts.

### Process for the manufacture of a formulation according to the present invention

The formulation of the present invention may be prepared according to the following procedure:
a) preparing an aqueous solution or colloidal solution of a protective colloid;
b) optionally adding at least a water-soluble excipient and/or adjuvant to the solution prepared in step a);
c) preparing a solution or dispersion of vitamin A and/or a derivate thereof, and optionally at least a fat-soluble adjuvant and/or excipient;
d) adding the antioxidants BHT and/or BHA to the solution or dispersion prepared in step c);
e) mixing the solutions prepared in step a) (or b)) and d) with each other;
f) homogenising the thus resulting mixture;
g) converting the nano-emulsion/dispersion obtained in step f) into a powder;
h) optionally further drying said powder obtained in step g).

Steps a) to h) of this process for the manufacture of the compositions of the present invention can be carried out in an according manner as disclosed for the preparation of matrix-based compositions of (fat-soluble) active ingredient and/or colorant compositions for enrichment, fortification and/or coloration of food, beverages, animal feed, cosmetics or pharmaceutical compositions, e.g. in EP-A 0 285 682, EP-A 0 347 751, EP-A 0 966 889, EP-A 1 066 761, EP-A 1 106 174, WO 98/15195, EP-A 0 937 412, EP-A 0 065 193 or the corresponding US 4,522,743, WO 02/102298, EP-A 1 300 394 and in EP-A 0 347 751, the contents of which are incorporated herein by reference.

This process may be performed at industrial scale, i.e. at a scale to obtain several 100 kg to several tons of product. If the process in the plant is carried out continuously several 100 tons of product may be obtained.

Details of this process are discussed in the following.

### Step a)

In step a) preferably an aqueous solution or suspension of a protective colloid (with the definition and the preferences as described above) having a dry mass content in the range of from 0.1 to 80 weight-%, preferably in the range of from 0.5 to 80 weight-%, is prepared.

Step a) is preferably carried out at a temperature in the range of from 20 to 80°C and at atmospheric pressure.

During step a) other water-soluble ingredients of the final composition may also be added.

### Step b)

Step b) is preferably carried out at the same temperature and at the same pressure as step a).

### Step c)

The vitamin A (derivative) and optional fat-soluble excipients and adjuvents (e.g. emulsifiers and stabilizers) are either used as such or dissolved or suspended in an oil and/or an (organic) solvent. Most preferably the vitamin A (derivative) and optionally the fat-soluble excipients and adjuvents is/are dissolved or suspended in oil such as corn oil and/or triglycerides, especially in corn oil and/or middle chain triglycerides (MCT).

Suitable organic solvents are halogenated aliphatic hydrocarbons, aliphatic ethers, aliphatic and cyclic carbonates, aliphatic esters and cyclic esters (lactones), aliphatic and cyclic ketones, aliphatic alcohols and mixtures thereof.

Examples of halogenated aliphatic hydrocarbons are mono- or polyhalogenated linear, branched or cyclic C₁- to C₁₅-alkanes. Especially preferred examples are mono- or polychlorinated or -brominated linear, branched or cyclic C₁- to C₁₅-alkanes. More preferred are mono- or polychlorinated linear, branched or cyclic C₁- to C₁₅-alkanes. Most preferred are methylene chloride and chloroform.

Examples of aliphatic esters and cyclic esters (lactones) are ethyl acetate, isopropyl acetate and n-butyl acetate; and γ-butyrolactone.

Examples of aliphatic and cyclic ketones are acetone, diethyl ketone and isobutyl methyl ketone; and cyclopentanone and isophorone.

Examples of cyclic carbonates are especially ethylene carbonate and propylene carbonate and mixtures thereof.

Examples of aliphatic ethers are dialkyl ethers, where the alkyl moiety has 1 to 4 carbon atoms. One preferred example is dimethyl ether.

Examples of aliphatic alcohols are ethanol, iso-propanol, propanol and butanol.

Furthermore any oil (triglycerides), orange oil, limonen or the like and water can be used as a solvent.

Step d) is preferably carried out at the same temperature and at the same pressure as step a) and/or step b).

### Step d)

The antioxidants 2,6-ditertbutyl-4-methylphenol (BHT) and 2-tert-butyl-4-hydroxyanisol (BHA) are usually added as such to the solution/dispersion prepared in step c). It may also be possible, however, to dissolve or suspend BHT and/or BHA in an oil and/or an (organic) solvent and then add it/them as suspension or solution to the solution prepared in step c). Both antioxidants may be added together or separately, optional as solution or suspension, to the solution prepared in step c).

### Step e)

Usually the vitamin A (derivative) or the solution or dispersion thereof containing the antioxidants BHT and BHA, respectively, is then added to the aqueous (colloidal) solution of the protective colloid with stirring.

### Step f)

For the homogenisation conventional technologies, such as high-pressure homogenisation, high shear emulsification (rotor-stator systems), micronisation, wet milling, microchanel emulsification, membrane emulsification or ultrasonification can be applied. Other techniques are e.g. disclosed in EP-A 0 937 412 (especially paragraphs [0008], [0014], [0015], [0022] to [0028]), EP-A 1 008 380 (especially paragraphs [0005], [0007], [0008], [0012], [0022], [0023] to [0039]) and in US 6,093,348 (especially column 2, line 24 to column 3, line 32; column 3, line 48 to 65; column 4, line 53 to column 6, line 60), the contents of which are incorporated herein by reference.

### Step g)

The so-obtained nano-emulsion/dispersion, which is an oil-in-water dispersion, can be converted after removal of the organic solvent (if present) into a solid composition, e.g. a dry powder, using any conventional technology such as spray drying, spray drying in combination with fluidised bed granulation (the latter technique commonly known as fluidised spray drying or FSD), or by a powder-catch technique (resulting in the formation of beadlets) whereby sprayed emulsion droplets are caught in a bed of an absorbent, such as starch, and subsequently dried.

### Step h)

The drying may be performed by any method known to the person skilled in the art.

Preferably the drying according to step h) is carried out in a fluidized bed. This is also the preferred drying mode if the process is an industrial process.

If the process is carried out in the batch mode, the drying is preferably carried out at a temperature in the range of from 30-70°C, preferably at a temperature in the range of from 35-65°C, even more preferably at a temperature in the range of from 38-64°C, most preferably at a temperature in the range of from 50-60°C. The drying at these temperature ranges may last between 20 - 90 minutes, preferably between 30 - 70 minutes, more preferably between 30 - 45 minutes.

A further object of the present invention is the use of an antioxidant selected from the group consisting of 2,6-ditertbutyl-4-methylphenol, 2-tert-butyl-4-hydroxyanisol and mixtures thereof to increase the stability of gelatine-free formulations of vitamin A and its derivatives. Preferably a mixture of BHT and BHA (preferably in a weight ratio of 1 : 1) is used.

Another object of the present invention is the use of an antioxidant selected from the group consisting of 2,6-ditertbutyl-4-methylphenol, 2-tert-butyl-4-hydroxyanisol and mixtures thereof to increase the stability of gelatine-free vitamin A tablets based on gelatine-free formulations of vitamin A and its derivatives. Preferably a mixture of BHT and BHA (preferably in a weight ratio of 1 : 1) is used.

## Claims

1. A tablet comprising
i) a carrier selected from the group consisting of calcium carbonate, microcrystalline cellulose, magnesium stearate, maltodextrin, and mixtures thereof; and
ii) a formulation, wherein the formulation comprises vitamin A and/or a derivate thereof, a protective colloid and an antioxidant selected from the group consisting of 2,6-ditertbutyl-4-methylphenol, 2-tert-butyl-4-hydroxyanisol and mixtures thereof, wherein the protective colloid is not gelatine.

2. The tablet according to claim 1, wherein the tablet is a multivitamin tablet.

3. The tablet according to claim 1 or 2 further comprising mineral salts.

4. The tablet according to any of the preceding claims, wherein the antioxidant is a mixture of 2,6-ditertbutyl-4-methylphenol and 2-tert-butyl-4-hydroxyanisol, preferably a mixture in a weight ratio of 1 : 1.

5. The tablet according to any of the preceding claims, wherein the vitamin A derivative is vitamin A acetate and/or vitamin A palmitate, preferably vitamin A acetate.

6. The tablet according to any of the preceding claims, wherein the tablet is gelatine-free.

7. Use of an antioxidant selected from the group consisting of 2,6-ditertbutyl-4-methylphenol, 2-tert-butyl-4-hydroxyanisol and mixtures thereof to increase the stability of gelatine-free vitamin A tablets based on gelatine-free formulations of vitamin A and derivatives thereof.

8. The use according to claim 7, wherein the antioxidant is a mixture of 2,6-ditertbutyl-4-methylphenol and 2-tert-butyl-4-hydroxyanisol, preferably a mixture in a weight ratio off : 1.

9. The use according to claim 7 and/or 8, wherein the vitamin A derivative is vitamin A acetate and/or vitamin A palmitate, preferably vitamin A acetate.

10. Use of an antioxidant selected from the group consisting of 2,6-ditertbutyl-4-methylphenol, 2-tert-butyl-4-hydroxyanisol and mixtures thereof to increase the stability of gelatine-free formulations of vitamin A and derivatives thereof.

11. The use according to claim 10, wherein the antioxidant is a mixture of 2,6-ditertbutyl-4-methylphenol and 2-tert-butyl-4-hydroxyanisol, preferably a mixture in a weight ratio of 1 : 1.

12. The use according to claim 10 and/or 11, wherein the vitamin A derivative is vitamin A acetate and/or vitamin A palmitate, preferably vitamin A acetate.

13. A formulation of vitamin A suitable for pharmaceutical applications comprising vitamin A and/or a derivate thereof, a protective colloid and an antioxidant selected from the group consisting of 2,6-ditertbutyl-4-methylphenol, 2-tert-butyl-4-hydroxyanisol and mixtures thereof, wherein the protective colloid is not gelatine.

14. The formulation according to claim 13, wherein the antioxidant is a mixture of 2,6-ditertbutyl-4-methylphenol and 2-tert-butyl-4-hydroxyanisol, preferably a mixture in a weight ratio of 1 : 1.

15. A process for the manufacture of a formulation according to claim 13 and/or 14 comprising the following steps:
a) preparing an aqueous solution or colloidal solution of a protective colloid;
b) optionally adding at least a water-soluble excipient and/or adjuvant to the solution prepared in step a);
c) preparing a solution or dispersion of vitamin A and/or a derivate thereof, and optionally at least a fat-soluble adjuvant and/or excipient;
d) adding the antioxidants 2,6-ditertbutyl-4-methylphenol and/or 2-tert-butyl-4-hydroxyanisol to the solution or dispersion prepared in step c);
e) mixing the solutions prepared in step a) (or b)) and d) with each other;
f) homogenising the thus resulting mixture;
g) converting the nano-emulsion/dispersion obtained in step f) into a powder;
h) optionally further drying said powder obtained in step g).
